# EUROPÄISCHE PATENTSCHRIFT

(11) **EP 0 942 691 B1**
(45) Veröffentlichungstag und Bekanntmachung des Hinweises auf die Patenterteilung: **04.05.2005**
(21) Anmeldenummer: 96938896.6
(22) Anmeldetag: 29.11.1996
(51) Int. Cl.: A61F 2/36

(54) **FEMURKOMPONENTE EINER HUFTGELENK-ENDOPROTHESE**
FEMUR COMPONENT OF A HIP JOINT ENDOPROSTHESIS
ELEMENT FEMUR D'UNE ENDOPROTHESE D'ARTICULATION DE LA HANCHE

(43) Veröffentlichungstag der Anmeldung: 22.09.1999
(73) Patentinhaber: Mathys Medizinaltechnik AG, 2544 Bettlach (CH)
(72) Erfinder: MOSER, Walter, D-3126 Kaufdorf (CH); COTTING, Anton, D-2540 Grenchen (CH)
(74) Vertreter: Lusuardi, Werther Giovanni, Dr.
(86) Internationale Anmeldenummer: PCT/CH1996/000421
(87) Internationale Veröffentlichungsnummer: WO 1998/023231

(56) Entgegenhaltungen:
- EP-A- 0 222 236
- EP-A- 0 378 044
- EP-A- 0 669 116
- DE-A- 3 505 997

## Beschreibung

Die Erfindung betrifft eine Femurkomponente einer Hüftgelenk-Endoprothese gemäss dem Oberbegriff des Patentanspruchs 1.

Aus dem Stand der Technik sind bereits solche Femurkomponenten bekannt, welche allerdings mit verschiedenen Nachteilen behaftet sind.

Bei unzementierten Femurprothesen für den Hüftgelenksersatz wird die primäre Stabilisierung des Femurschaftes durch einen Form- und Kraftschluss mit dem umgebenden Knochen erzielt. Die Formgebung des Femurschaftes sollte so gestaltet sein, dass sich bei Belastung eine Verkeilung im knöchernen Lager ergibt. Besonders in der ersten Belastungsphase, wo gewisse Setzbewegungen des Femurschaftes wahrscheinlich sind, muss durch eine entsprechende Formgebung eine sichere Primärfixation erzielt werden. Im Falle von Setzbewegungen muss eine erneute Stabilisierung durch eine entsprechende Gestaltung der Geometrie gewährleistet werden. Ohne ausreichende primäre Stabilität, kommt es bei einer Belastung zu sich wiederholenden Bewegungen in der Grenzfläche zwischen Femurschaft und Knochen, was eine sichere Einheilung des Implantats verhindert. Unter der Voraussetzung einer sicheren primären Verankerung kann das Implantat dagegen im Heilungsprozess knöchern umwachsen werden, was eine Voraussetzung für ein gutes Langzeitresultat bietet.

Vorzugsweise wird die primäre Fixation im oberen Teil des Prothesenschaftes, der von spongiösem Knochen umgeben ist gesucht. Im dort vorhandenen grossen Knochenvolumen kann eine grossflächig Abstützung erzielt werden. Die Krafteinleitung über diesen Bereich hat sich aus biomechanisch-klinischer Sicht als vorteilhaft erwiesen.

So ist im Markt beispielsweise ein Femurschaft- bekannt, bei welchem sich der zur Verankerung in der spongiösen Knochenstruktur bestimmte, proximale Schaftteil von lateral nach medial kontinuierlich konisch verjüngt, um bei einem Nachgeben des Knochens in medialer Richtung eine erneute, selbständige Verklemmung zu erhalten. Die Region des Trochanter Major mit dem Verankerungsraum zeigt im Querschnitt betrachtet jedoch nicht eine dreieckige, bzw. trapezförmige, sondern eher eine ovale Form. Die Nachteile dieses bekannten Femurschafts bestehen somit darin, dass die auf der lateralen Seite stark verbreiterte proximale Schaftpartie den Knochen sprengen kann. Zusätzlich wirken bei diesem bekannten Femurschaft die massiv ausgebildeten Rippen bei der Verdrängung des Knochenvolumens druckerhöhend, was einen weiteren Beitrag zur Sprengwirkung erzeugen kann.

Betrachtet man einen Längsschnitt des proximalen Femurs mit einem darin eingesetzten bekannten Femurschaft, so erkennt man, dass die Spongiosa nicht scharf abgegrenzt auf die Trochanter region begrenzt ist, sondern sich teilweise bis in die Region des diaphysären Knochenrohres fortsetzt. Es sollte aber angestrebt werden, möglichst viel von dieser Knochenstruktur zur Lasteinleitung zu verwenden. Die an einer Stelle des bekannten Femurschaftes angesetzten Rippen erfüllen jedoch auf Grund der wenig differenzierten Anordnung diese Anforderungen nicht optimal. Der Ansatzpunkt und die Ausdehnung der Rippen am Schaft sollte so gestaltet sein, dass möglichst viel des spongiösen Volumens des proximalen Femur für die Verankerung genutzt wird.

Aus der EP-A-222236 SULZER ist eine Femurkomponente gemäß dem Oberbegriff von Anspruch 1 bekannt mit Längsrippen, deren Umhüllende eine ellipsoide Form aufweist. Nachteilig bei diesem bekannten Schaft ist jedoch der Umstand, dass nur der sich prismatisch erweiternde, proximale Teil des Schaftes mit Rippen versehen ist.

Hier will die Erfindung Abhilfe schaffen. Der Erfindung liegt das Problem zugrunde eine Femurkomponente einer Hüftgelenk-Endoprothese zu schaffen, welche optimal der Spongiosa architektur im proximalen Teil des Femurs entspricht und zu einer möglichst stabilen, zementlosen, primären Verankerung des Schaftes im Femur führt, um auf diese Weise gute Voraussetzungen für eine knöcherne Einheilung zu bieten.

Die Erfindung löst die gestellte Aufgabe mit einer Femurkomponente, welche die Merkmale des Anspruchs 1 aufweist.

Dank der Drachenviereck - Form des proximalen Schaftteils ist der Vorteil erzielbar, dass sich der Prothesenschaft sowohl nach lateral als auch nach medial bei einer allfälligen Setzbewegung automatisch erneut verkeilen kann. Durch die dem Querschnitt des proximalen Femurs angepasste Umhüllende der Rippen, wird die Gefahr des Sprengens des proximalen Femurs durch direkten Drück auf den harten kortikalen Knochen vermindert.

Bei einer bevorzugten Weiterbildung der Erfindung, bei welcher die Rippen im Querschnitt dreieckförmig ausgebildet sind, können diese leicht in das spongiöse Knochenvolumen eindringen, was den Druck beim Einschlagprozess vermindert. Durch den vorzugsweise gesamthaft konischen Verlauf der dreiecksförmigen Rippen wird eine zusätzliche Verkeilwirkung erzielt, welche bei der Verwendung von rechteckförmigen Rippen - wie beim Stand der Technik - fehlt.

Die gerade Schaftform mit der in Richtung der Schaftachse verlaufenden Rippen mit zunehmender Höhe nach proximal erlaubt ein sicheres Positionieren und Einschlagen des Femurschaftes, geführt durch die selbstschneidenden Rippen. Sind die Rippen demgegenüber teilweise oder ganz in einem Winkel zur Schaftachse angeordnet so kann beim Setzvorgang des Femurschaftes kein die Rippen umfassender Sitz erzielt werden. Durch den längs des Schaftes unterschiedlichen Rippenansatz wird das Spongiosavolumen homogener beansprucht, als bei bekannten Schäften mit Rippen, die auf einer bestimmten Höhe angesetzt sind, und deren Höhe von dort aus kontinuierlich zunimmt.

Eine weitere bevorzugte Weiterbildung besteht darin, dass die Kämme der Längsrippen einen Winkel δ/2 von mindestens 1°, vorzugsweise mindestens 2° zur Symmetrieebene einschliessen. Die einzelnen Kämme der Längsrippen schliessen dabei unterschiedliche Winkel δ/2 im Bereich von 3° bis 8° zur Symmetrieebene ein, wobei vorzugsweise die gegen die laterale Seite und mediale Seite gelegenen Längsrippen einen grösseren Winkel δ/2 als die dazwischenliegenden Längsrippen aufweisen. Eine solche Rippengeometrie erzeugt einen funktionellen Reiz auf den umgebenden Knochen, der durch eine stumpfe Rippenform - wie beim Stand der Technik - nicht gegeben ist. Dieser funktionelle Reiz führt im beanspruchten Bereich zu einer Knochenneubildung mit anschliessender Verdichtung und somit zu einer knöchernen Einheilung. Im Bereich der Täler dieser Rippenstruktur kann sich die Blutversorgung des neu generierten Knochens optimal ausbilden.

Zweckmässigerweise bildet die anteriore Fläche mit der posterioren Fläche einen zum distalen Teil hin zulaufenden Keil mit der Symmetrieebene als Mittelebene, dessen Keilwinkel ε im Bereich von 0,5° bis 3,0°, vorzugsweise im Bereich von 1,0° - 2,0° liegt. Durch diese Geometrie setzt sich die Verkeilwirkung auch längs des oberen Schaftbereiches fort. bei einer etwa fällig werdenden Reoperation eines fest integrierten Schaftes kann bei allseitig konischer Geometrie, also auch proximal im Zwischenrippenbereich, der Schaft leichter ausgeschlagen werden, als bei nicht konischer Geometrie.

In einem zur Symmetrieebene des Schaftes orthogonalen Schnitt stellt die Umhüllende der Kämme der Längsrippen ein Drachenviereck dar.

Das Drachenviereck sollte gegen die mediale Seite hin einen Eckwinkel α von grösser als 10°, vorzugsweise grösser als 12° aufweisen. Der Eckwinkel α sollte zudem kleiner als 22°, vorzugsweise kleiner als 20°sein.
Das Drachenviereck sollte gegen die laterale Seite hin einen Eckwinkel β von grösser als 8°, vorzugsweise grösser als 9° aufweisen. Der Eckwinkel β sollte zudem kleiner als 45°, vorzugsweise kleiner als 40° sein.

Die Kämme der Längsrippen sind zweckmässigerweise scharf ausgebildet und in einem zur Symmetrieebene orthogonalen Schnitt vorzugsweise dreieckig ausgebildet. Die Längsrippen können beispielsweise als dreiseitige Pyramiden ausgebildet sein wobei die Pyramidenspitzen gegen distal gerichtet sind. Die Kämme der Längsrippen können aber auch abgerundet ausgebildet sein und in einem zur Symmetrieebene orthogonalen Schnitt vorzugsweise halbkreisförmig ausgebildet sein. Zu vermeiden sind hingegen gleichmässig dicke Längsrippen, die ein rechteckiges Profil aufweisen.

Die Breite der Längsrippen sollte zweckmässigerweise von proximal nach distal abnehmen, vorzugsweise in kontinuierlicher Weise. Das gleich gilt für die Höhe der Längsrippen, welche von proximal nach distal abnehmen sollte, vorzugsweise in kontinuierlicher Weise.

Überraschenderweise haben sich besonders gute klinische Resultate eingestellt, wenn mindestens eine der Längsrippen bis in die distale Hälfte des Schaftes verläuft, weil dadurch eine erhöhte Primärstabilität erreicht werden kann und sich die Knochenneubildung, bzw. Knochenumbildung ausgehend von dieser Verankerungsregion im Sinne einer Osteokonduktion nach proximal fortsetzt.

Weitere Vorteile konnten mit Ausführungsformen erzielt werden, bei welchen der Schaft kragenlos ausgebildet ist und der Schaft in einem zur Symmetrieebene orthogonalen Schnitt einen im wesentlichen rechteckigen Querschnitt aufweist.

Die Erfindung und Weiterbildungen der Erfindung werden im folgenden anhand der teilweise schematischen Darstellungen mehrerer Ausführungsbeispiele noch näher erläutert.

Es zeigen:
Fig. 1, eine Ansicht der erfindungsgemässen Femurkomponente von anterior mit zwei Querschnittsprofilen;
Fig. 2 eine Ansicht der Femurkomponente nach Fig. 1 von lateral;
Fig. 3 einen Schnitt durch die Femurkomponente nach Fig. 1 längs der Linie III-III; und

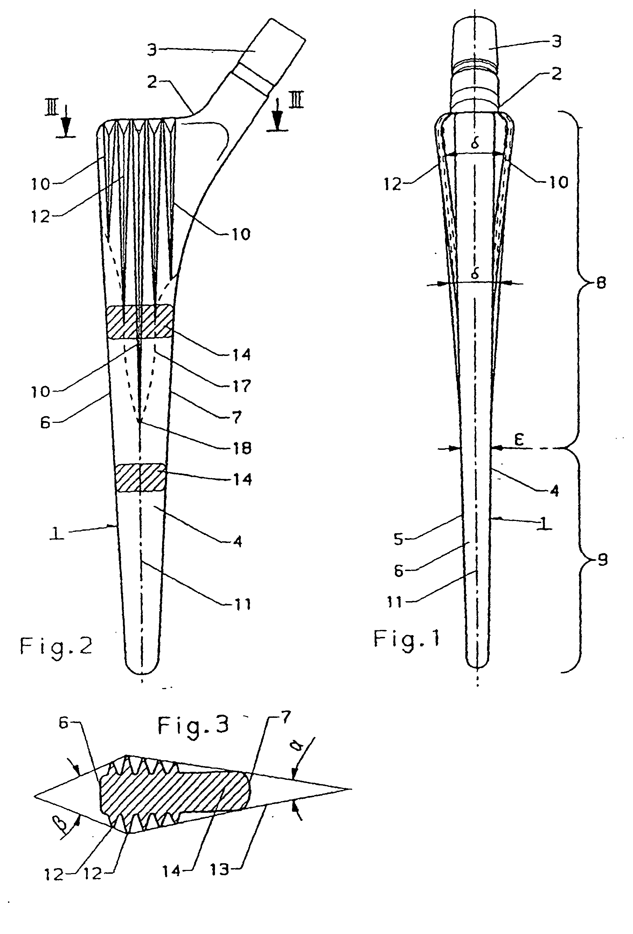

Die in den Fig. 1 - 3 dargestellte Femurkomponente einer Hüftgelenk-Endoprothese umfasst im wesentlichen einen zur Verankerung in der Markhöhle des Femur bestimmten, kragenlosen Schaft 1 mit einem distalen Teil 9 und einem proximalen Teil 8, an welchem eine Halspartie 2 mit einem Zapfen 3 zur Aufnahme eines Gelenkkopfes oder einem mit der Halspartie 2 fest verbundenen Gelenkkopf anschliesst. Der Schaft 1 weist eine anteriore Fläche 4, eine posteriore Fläche 5, eine laterale Seite 6, eine mediale Seite 7 und eine Symmetrieebene 11 auf, wobei letztere mit der Zeichenebene von Fig. 1 identisch ist. In einem zur Symmetrieebene 11 orthogonalen Schnitt, weist der Schaft einen im wesentlichen rechteckigen Querschnitt 14 auf.

Auf der anterioren Fläche 4 und der posterioren Fläche 5 sind im proximalen Teil 8 des Schaftes 1 von proximal nach distal verlaufende Längsrippen 10 angebracht. Die Kämme 12 der Längsrippen 10 schliessen je nach Lage einen Winkel δ/2 von 3° bis 8° zur Symmetrieebene 11 ein. Die gegen die laterale Seite 6 und mediale Seite 7 gelegenen Längsrippen 10 weisen dabei einen grösseren Winkel δ/2 als die dazwischenliegenden Längsrippen 10 auf. Die einzelnen Längsrippen 10 weisen zudem eine unterschiedliche Länge auf, wobei vorzugsweise die gegen die laterale Seite 6 und mediale Seite 7 gelegenen Längsrippen 10 kürzer sind als die dazwischenliegenden Längsrippen 10. Die Verbindungslinie 17 der in die anterioren und posterioren Flächen 4,5 übergehenden Ansatzstellen 18 der Längsrippen 10 liegen dabei nicht auf einer Geraden, sondern auf einer parabelförmigen oder ellipsenförmigen Kurve.

Die Umhüllende der Kämme 12 der Längsrippen 10 bildet ein Drachenviereck, das sich sowohl gegen die laterale Seite 6, als auch gegen die mediale Seite 7 hin verjüngt.

Die anteriore Fläche 4 bildet im weiteren mit der posterioren Fläche 5 einen zum distalen Teil 9 hin zulaufenden Keil 4,5 mit der Symmetrieebene 11 als Mittelebene, wobei der Keilwinkel ε des Keiles 4,5 den Wert von 0,5° aufweist.

Wie in Fig. 3 dargestellt bildet die Umhüllende der Kämme 12 der Längsrippen 10 in einem zur Symmetrieebene 11 orthogonalen Schnitt ein Drachenviereck 13, wobei die kurzen Seiten gegen lateral und die langen Seiten gegen medial gerichtet sind.

Gegen die mediale Seite 7 hin weist das Drachenviereck 13 einen Eckwinkel α von 12° bis 20° auf; gegen die laterale Seite 6 hin einen Eckwinkel β von 9° bis 44°.

Die Kämme 12 der Längsrippen 10 sind scharf ausgebildet und weisen in einem zur Symmetrieebene 11 orthogonalen Schnitt eine dreieckiges Profil auf. Die Breite und Höhe der Längsrippen 10 nimmt von proximal nach distal kontinuierlicher ab. Die Längsrippen 12 sind somit als dreiseitige Pyramiden ausgebildet, wobei die Pyramidenspitze gegen distal gerichtet ist. Die zwischen den einzelnen Längsrippen 10 liegenden Täler verengen sich somit vom distalen Teil 9 zum proximalen Teil 8 hin.
Wie aus Fig. 1 ersichtlich reicht eine der Längsrippen 12 (die mittlere) bis in die distale Hälfte des Schaftes 1, was die Primärstabilität des implantierten Schaftes erhöht.

## Patentansprüche

1. Femurkomponente einer Hüftgelenk-Endoprothese mit einem zur Verankerung in der Markhöhle des Femur bestimmten Schaft (1) mit einem distalen Teil (9) und einem proximalen Teil (8), an welchem eine Halspartie (2) mit einem Zapfen (3) zur Aufnahme eines Gelenkkopfes oder einem mit der Halspartie (2) fest verbundenen Gelenkkopf anschliesst, wobei der Schaft (1) eine anteriore Fläche (4), eine posteriore Fläche (5), eine laterale Seite (6), eine mediale Seite (7) und eine Symmetrieebene (11) aufweist, wobei auf der anterioren Fläche (4) und posterioren Fläche (5) im proximalen Teil (8) des Schaftes (1) von proximal nach distal verlaufende Längsrippen (10) angebracht sind und sich die Umhüllende der Kämme (12) der Längsrippen (10) sowohl gegen die laterale Seite (6) als auch gegen die mediale Seite (7) hin verjüngt, und wobei die Höhe der Längsrippen (10) von proximal nach distal abnimmt,
**dadurch gekennzeichnet, dass**
die Umhüllende der Kämme (12) der Längsrippen (10) in einem zur Symmetrieebene (11) orthogonalen Schnitt ein Drachenviereck (13) bildet.

2. Femurkomponente nach Anspruch 1, **dadurch gekennzeichnet, dass** die Kämme (12) der Längsrippen (10) einen Winkel δ/2 von mindestens 1°, vorzugsweise von mindestens 2° zur Symmetrieebene (11) einschliessen.

3. Femurkomponente nach Anspruch 2, **dadurch gekennzeichnet, dass** die einzelnen Kämme (12) der Längsrippen (10) unterschiedliche Winkel δ/2 im Bereich von 3° bis 8° zur Symmetrieebene (11) einschliessen, wobei vorzugsweise die gegen die laterale Seite (6) und mediale Seite (7) gelegenen Längsrippen (10) einen grösseren Winkel δ/2 als die dazwischenliegenden Längsrippen (10) aufweisen.

4. Femurkomponente nach einem der Ansprüche 1 bis 3, **dadurch gekennzeichnet, dass** die anteriore Fläche (4) mit der posterioren Fläche (5) einen zum distalen Teil (9) hin zulaufenden Keil (4,5) mit der Symmetrieebene (11) als Mittelebene bildet, der einen Keilwinkel ε im Bereich von 0,5° bis 3,0°, vorzugsweise im Bereich von 1,0° - 2,0° einschliesst;

5. Femurkomponente nach einem der Ansprüche 1 bis 4, **dadurch gekennzeichnet, dass** die einzelnen Längsrippen (10) eine unterschiedliche Länge aufweisen, wobei vorzugsweise die gegen die laterale Seite (6) und mediale Seite (7) gelegenen Längsrippen (10) kürzer sind als die dazwischenliegenden Längsrippen (10).

6. Femurkomponente nach einem der Ansprüche 1 bis 5, **dadurch gekennzeichnet, dass** die Kämme (12) der Längsrippen (10) scharf ausgebildet sind und in einem zur Symmetrieebene (11) orthogonalen Schnitt vorzugsweise dreieckig ausgebildet sind.

7. Femurkomponente nach einem der Ansprüche 1 bis 5, **dadurch gekennzeichnet, dass** die Kämme (12) der Längsrippen (10) abgerundet ausgebildet ist und in einem zur Symmetrieebene (11) orthogonalen Schnitt vorzugsweise halbkreisförmig ausgebildet sind.

8. Femurkomponente nach einem der Ansprüche 1 bis 7, **dadurch gekennzeichnet, dass** die Breite der Längsrippen (10) in kontinuierlicher Weise von proximal nach distal abnimmt.

9. Femurkomponente nach einem der Ansprüche 1 bis 7, **dadurch gekennzeichnet, dass** die Höhe der Längsrippen (10) in kontinuierlicher Weise von proximal nach distal abnimmt.

10. Femurkomponente nach einem der Ansprüche 1 bis 9, **dadurch gekennzeichnet, dass** das Drachenviereck (13) gegen die mediale Seite (7) hin einen Eckwinkel α von grösser als 10° aufweist.

11. Femurkomponente nach Anspruch 10, **dadurch gekennzeichnet, dass** der Eckwinkel α grösser als 12° ist.

12. Femurkomponente nach einem der Ansprüche 1 bis 11, **dadurch gekennzeichnet, dass** das Drachenviereck (13) gegen die mediale Seite (7) hin einen Eckwinkel α von kleiner als 22° aufweist.

13. Femurkomponente nach Anspruch 12, **dadurch gekennzeichnet, dass** der Eckwinkel α kleiner als 20° ist.

14. Femurkomponente nach einem der Ansprüche 1 bis 13, **dadurch gekennzeichnet, dass** das Drachenviereck (13) gegen die laterale Seite (6) hin einen Eckwinkel β von grösser als 8°, vorzugsweise grösser als 9° aufweist.

15. Femurkomponente nach einem der Ansprüche 1 bis 14, **dadurch gekennzeichnet, dass** das Drachenviereck (13) gegen die laterale Seite (6) hin einen Eckwinkel β von kleiner als 45°, vorzugsweise kleiner als 40° aufweist.

16. Femurkomponente nach einem der Ansprüche 1 bis 15, **dadurch gekennzeichnet, dass** der Schaft (1) kragenlos ausgebildet ist.

17. Femurkomponente nach einem der Ansprüche 1 bis 16, **dadurch gekennzeichnet, dass** der Schaft (1) in einem zur Symmetrieebene (11) orthogonalen Schnitt einen im wesentlichen rechteckigen Querschnitt (14) aufweist.

18. Femurkomponente nach einem der Ansprüche 1 bis 17, **dadurch gekennzeichnet, dass** die Längsrippen (12) als dreiseitige Pyramiden ausgebildet sind, wobei die Pyramidenspitze gegen distal gerichtet ist.

19. Femurkomponente nach einem der Ansprüche 1 bis 18, **dadurch gekennzeichnet, dass** die Verbindungslinie (17) der in die anterioren und posterioren Flächen (4,5) übergehenden Ansatzstellen (18) der Längsrippen (10) nicht auf einer Geraden liegen und vorzugsweise auf einer parabelförmigen oder ellipsenförmigen Kurve liegen.

20. Femurkomponente nach einem der Ansprüche 1 bis 19, **dadurch gekennzeichnet, dass** die zwischen den einzelnen Längsrippen (10) liegenden Täler sich vom distalen Teil (9) zum proximalen Teil (8) hin verengen.

## Claims

1. A femur component for a hip-joint endoprosthesis fitted with a shaft (1) to be anchored in the femur's marrow cavity and comprising a distal segment (9) and a proximal segment (8) adjoined by a neck (2) with a stub (3) to receive a hinge head or with a hinge head firmly adjoining the neck (2), further a posterior surface (5) a lateral side (6), a mesial side (7) and a plane of symmetry (11), longitudinal ribs (10) running in the proximal-to-distal direction being present on the anterior surface (4) and on the posterior surface (5) in the proximal segment (8) of the shaft (1), and the envelope curve of the combs (12) of the longitudinal ribs (10) tapering both in the direction of the lateral side (6) and in the direction of the mesial side (7), and whereby the height of the longitudinal ribs (10) decreases in the proximal-to-distal direction,
**characterized in that**
when seen in a section orthogonal to the plane of symmetry (11), the envelope curve of the combs (12) of the longitudinal ribs (10) forms a kite quadrilateral (13).

2. Femur component as claimed in claim 1, **characterized in that** the combs (12) of the longitudinal ribs (10) subtend an angle δ/2 of at least 1°, preferably at least 2° with the plane of symmetry (11).

3. Femur component as claimed in claim 2, **characterized in that** the combs (12) of the longitudinal ribs (10) subtend an angle δ/2 in the range of 3° to 8° with the plane of symmetry (11), preferably the longitudinal ribs (10) situated near the lateral side (6) and the mesial side (7) subtending a larger angle δ/2 than do the longitudinal ribs in-between.

4. Femur components as claimed in one of claims 1 through 3, **characterized in that** the anterior surface (4) jointly with the posterior surface (5) forms a wedge (4, 5) tapering toward the distal segment (9), where the plane of symmetry (11) is the center plane, said wedge subtending an angle ε in the range of 0.5° to 3.0° and preferably in the range of 1.0° to 2.0°.

5. Femur components as claimed in one of claims 1 through 4, **characterized in that** the individual longitudinal ribs (10) differ in length between themselves, preferably the longitudinal ribs (10) situated near the lateral side (6) and the mesial side (7) being shorter than the longitudinal ribs (10) in-between.

6. Femur component as claimed in one of claims 1 through 5, **characterized in that** the combs (12) of the longitudinal ribs (10) are sharp and preferably, when seen in a section orthogonal to the plane of symmetry (11), are triangular.

7. Femur component as claimed in one of claims 1 through 5, **characterized in that** the combs (12) of the longitudinal ribs (10) are rounded and, as seen in a section orthogonal to the plane of symmetry (11), preferably are semicircular.

8. Femur component as claimed in one of claims 1 through 7, **characterized in that** the width of the longitudinal ribs (10) decreases preferably continuously in the proximal-to-distal direction.

9. Femur component as claimed in one of claims 1 through 7, **characterized in that** the height of the longitudinal ribs (10) decreases preferably continuously in the proximal-to-distal direction.

10. Femur component as claimed in one of claims 1 through 9, **characterized in that** the kite quadrilateral (13) subtends an inside angle α larger than 10° with the mesial side (7).

11. Femur component as claimed in claim 10, **characterized in that** the kite quadrilateral (13) subtends an inside angle α larger than 12° with the mesial side (7).

12. Femur component as claimed in one of claims 1 through 11, **characterized in that** the kite quadrilateral (13) subtends an inside angle α less than 22° with the mesial side (7).

13. Femur component as claimed in claim 12, **characterized in that** the kite quadrilateral (13) subtends an inside angle α less than 20° with the mesial side (7).

14. Femur component as claimed in one of claims 1 through 13, **characterized in that** the kite quadrilateral (13) subtends an inside angle α larger than 8°, preferably larger than 9°, with the lateral side (6).

15. Femur component as claimed in one of claims 1 through 14, **characterized in that** the kite quadrilateral (13) subtends an inside angle β less than 45°, preferably less than 40°, with the lateral side (6).

16. Femur component as claimed in one of claims 1 through 15, **characterized in that** the shaft (1) is without collar.

17. Femur component as claimed in one of claims 1 through 16, **characterized in that** the shaft (1), when seen in a section orthogonal to the plane of symmetry (11) is essentially of rectangular cross-section (14).

18. Femur component as claimed in one of claims 1 through 17, **characterized in that** the longitudinal ribs (12) are in the form of three-sided pyramids of which the vertices point distally.

19. Femur component as claimed in one of claims 1 through 18, **characterized in that** the connection line (17) of the onset sites (18) of the longitudinal ribs (10) merging into the anterior and posterior surfaces (4, 5) are not rectilinear and preferably are on a parabolic or elliptic curve.

20. Femur component as claimed in one of claims 1 through 19, **characterized in that** the troughs situated between the individual longitudinal ribs (10) constrict from the distal segment (9) toward the proximal segment (8).

## Revendications

1. Composant fémoral d'une endoprothèse de l'articulation de la hanche comprenant un corps (1) destiné à être ancré dans la cavité médullaire du fémur et comportant une partie distale (9) et une partie proximale (8), laquelle est suivie d'une partie (2) constituant le col et pourvue soit d'un tenon (3) destiné à recevoir une tête fémorale, soit d'une tête fémorale reliée de manière solidaire à la partie (2) constituant le col, le corps (1) présentant une surface antérieure (4), une surface postérieure (5), un coté latéral (6), un côté médial (7) et un plan de symétrie (11), des nervures longitudinales (10) s'étendant, dans la partie proximale (8) du corps (1), sur la surface antérieure (4) et sur la surface postérieure (5), et ce depuis la partie proximale vers la partie distale, la ligne enveloppante des arêtes (12) des nervures longitudinales (10) allant en s'amincissant à la fois du côté latéral (6) et du côté médial (7), et la hauteur des nervures longitudinales (10) allant en diminuant depuis la partie proximale vers la partie distale,
**caractérisé en ce que**
la ligne enveloppante des arêtes (12) des nervures longitudinales (10) forme, dans une coupe orthogonale au plan de symétrie (11), un rhomboïde (13).

2. Composant fémoral selon la revendication 1, **caractérisé en ce que** les arêtes (12) des nervures longitudinales (10) forment un angle δ/2 d'au moins 1°, de préférence d'au moins 2° par rapport au plan de symétrie (11).

3. Composant fémoral selon la revendication 2, **caractérisé en ce que** les arêtes (12) des nervures longitudinales (10) forment, chacune, des angles différents δ/2 compris entre 3° et 8° par rapport au plan de symétrie (11), les nervures longitudinales situées du côté latéral et du côté médial présentant de préférence un angle δ/2 supérieur à celui des nervures longitudinales (10) situées entre celles-ci.

4. Composant fémoral selon l'une des revendications 1 à 3, **caractérisé en ce que** la surface antérieure (4) forme avec la surface postérieure (5) un coin (4,5) allant en s'amincissant vers la partie distale (9) et dont le plan central est le plan de symétrie (11), lequel forme un angle de coin ε compris entre 0,5° et 3,0°, de préférence entre 1,0° et 2,0°.

5. Composant fémoral selon l'une des revendications 1 à 4, **caractérisé en ce que** les nervures longitudinales (10) présentent, chacune, une longueur différente, les nervures longitudinales (10) situées du côté latéral (6) et du côté médial (7) étant de préférence plus courtes que les nervures longitudinales (10) situées entre celles-ci.

6. Composant fémoral selon l'une des revendications 1 à 5, **caractérisé en ce que** les arêtes (12) des nervures longitudinales (10) sont des arêtes coupantes et sont réalisées, dans une coupe orthogonale au plan de symétrie (11), de préférence en forme de triangle.

7. Composant fémoral selon l'une des revendications 1 à 5, **caractérisé en ce que** les arêtes (12) des nervures longitudinales (10) sont de forme arrondie et sont réalisées, dans une coupe orthogonale au plan de symétrie (11), de préférence en forme de demi-cercle.

8. Composant fémoral selon l'une des revendications 1 à 7, **caractérisé en ce que** la largeur des nervures longitudinales (10) diminue de manière continue depuis la partie proximale vers la partie distale.

9. Composant fémoral selon l'une des revendications 1 à 7, **caractérisé en ce que** la hauteur des nervures longitudinales (10) diminue de manière continue depuis la partie proximale vers la partie distale.

10. Composant fémoral selon l'une des revendications 1 à 9, **caractérisé en ce que** le rhomboïde (13) présente du côté médial (7) un angle α supérieur à 10°.

11. Composant fémoral selon la revendication 10, **caractérisé en ce que** l'angle α est supérieur à 12°.

12. Composant fémoral selon l'une des revendications 1 à 11, **caractérisé en ce que** le rhomboïde (13) présente du côté médial (7) un angle α inférieur à 22°.

13. Composant fémoral selon la revendication 12, **caractérisé en ce que** l'angle α est inférieur à 20°.

14. Composant fémoral selon l'une des revendications 1 à 13, **caractérisé en ce que** le rhomboïde (13) présente du côté latéral (6) un angle β supérieur à 8°, de préférence supérieur à 9°.

15. Composant fémoral selon l'une des revendications 1 à 14, **caractérisé en ce que** le rhomboïde (13) présente du côté latéral (6) un angle β inférieur à 45°, de préférence inférieur à 40°.

16. Composant fémoral selon l'une des revendications 1 à 15, **caractérisé en ce que** le corps (1) est dépourvu de collerette.

17. Composant fémoral selon l'une des revendications 1 à 16, **caractérisé en ce que** le corps (1) présente, dans une coupe orthogonale au plan de symétrie (11), une section transversale (14) essentiellement rectangulaire.

18. Composant fémoral selon l'une des revendications 1 à 17, **caractérisé en ce que** les nervures longitudinales 10 sont réalisées sous forme de pyramides à trois côtés, le sommet de chaque pyramide étant orienté vers la partie distale.

19. Composant fémoral selon l'une des revendications 1 à 18, **caractérisé en ce que** la ligne (17) reliant les points de départ (18) des nervures longitudinales (10) où ces dernières se confondent avec les surfaces antérieure et postérieure (4,5) ne se situe pas sur une droite mais sur une courbe en forme de parabole ou d'ellipse.

20. Composant fémoral selon l'une des revendications 1 à 19, **caractérisé en ce que** les zones creuses situées entre les différentes nervures longitudinales (10) vont en s'amincissant depuis la partie distale (9) vers la partie proximale (8).
